# EUROPEAN PATENT APPLICATION

(11) **EP 2 806 030 A1**
(43) Date of publication of application: **26.11.2014**
(21) Application number: 14168058.7
(22) Date of filing: 13.05.2014
(51) Int. Cl.: C12P 21/02

(54) **A process for producing lipase**

(30) Priority: 20.05.2013 MY PI2013700818
(71) Applicant: Universiti Putra Malaysia, Selangor (MY)
(72) Inventor: Raja Abdul Rahman, Raja Noor Zaliha, 43400 Serdang, Selangor (MY); Salleh, Abu Bakar, 43400 Serdang, Selangor (MY); Basri, Mahiran, 43400 Serdang, Selangor (MY); Mohd Nizar, Hafizal, 43400 Serdang, Selangor (MY)
(74) Representative: Hartvichova, Katerina

(57) **Abstract**

A process for producing lipase by microorganisms, preferably on the industrial scale, wherein *Escherichia coli* microorganism harbouring lipase, preferably thermostable lipase, is fermented under batch or fed batch conditions, in a Luria Bertani (LB) broth supplemented with citric acid, (NH₄)₂SO₄, Na₂HPO₄.12H₂O, MgCl₂, NH₄Cl and glucose, using a trace metal solution containing hydrochloric acid, FeSO₄.7H₂O, ZnSO₄.7H₂O, CaCl₂.H₂O, MnSO₄.5H₂O, CuSO₄.5H₂O, AlCl.6H₂O, and H₃BO₃, and a feeding nutrient solution containing glucose, yeast extract, MgSO₄.H₂O, KH₂PO₄, Citric Acid, (NH₄)₂SO₄, Na₂HPO₄.12H₂O, MgCl₂, and NH₄Cl, at dissolved oxygen tension of 30-50 %, at temperature of 30-40 °C, while maintaining the pH between 6 and 8, with a glucose content of 15-25 g/L, with isopropylthiogalactoside.

## Description

### Field of Art

The present invention relates to a process for producing lipase by microorganisms on the industrial scale.

### Background Art

Lipases or triacylglycerol acylhydrolases EC 3.1.1.3 are a class of enzymes that catalyze the hydrolysis of water-insoluble triacylglycerides to di- and mono-acylglycerides, free fatty acids and glycerol. This hydrolysis is apparently equilibrium, however, the direction of the reaction can be changed to ester synthesis by modification of the reaction conditions.

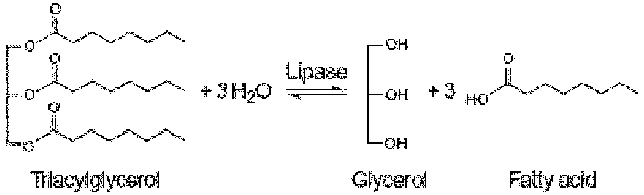

Lipases are widely distributed throughout animals, plants and microorganisms. Nevertheless, lipases originated from microorganisms, mainly fungal and bacterial lipases represent the most widely used class of enzymes in biotechnological applications and organic chemistry. As most of microbial lipases reported are extracellular, therefore these enzymes are easily obtained and can be produced in large quantities. Moreover, microbial lipases are quite stable under non-natural conditions such as high temperatures and nonaqueous organic solvents employed in many applications, such as detergents, dairy industry or sewage treatment. Apart from their stability, microbial lipases too display a broad synthetic potential, as well as their inexpensive manufacturing. The versatility of enzymic properties and substrate specificity have made microbial lipases commercially available indeed.

The lipases can be produced by fermentation of microorganisms carrying the lipase gene.

Fermentation can be carried out as batch, continuous and fed batch processes. The mode of operation is widely applied and depends on the type of product being produced.

Batch culture is closed culture system contain an initial, limited amount of nutrient. The inoculate culture will pass through a number of phases. After inoculation there is period which no growth appears, this period is referred as lag phase and be able to be adaptation period. In commercial process the length of lag phase is reduced as much as possible. Following the period which the growth rate of the cell is progressively increase and cell grow with maximum rate: this period known as the log or exponential rates. A stationary phase in batch culture is that point where the growth rate has declined to zero. However, the stationary phase is a misnomer in terms of the physiology of the organism, the population are still metabolically active during this phase and may produce products called secondary metabolites, which are not produced during the exponential phase.

The batch is important stage to understand the early process of fermentation for recombinant *E. coli* BL21(DE3)plysS(pGEX/T1) in the preferred working volume in scale up process. Beginning the research with batch culture can give a whole observation and a acceptable environmental parameter before proceeded to the fed batch process. The result from batch culture can become the comparison factor for the fed batch process where the percentage of yield can be determined.

Fed batch culture is a batch culture that is fed continuously, or sequently, with medium, without the removal of culture liquid. A fed batch culture is established initially in batch mode and is then fed accordingly to one of the following feed strategies:
i. The same medium used to establish the batch culture is added, resulting in an increase in volume.
ii. A solution of the limiting substrate at the same concentration as that in the initial medium is added, resulting in an increase in volume.
iii. A concentrated solution of limiting substrate is added at the rate less than (i) and (ii), resulting in an increase in volume.
iv. A very concentrated solution of the limiting substrate is added at the rate less than in (i) and (ii) and (iii), resulting in an significant increase in volume.

Fed batch system employing strategies (i) and (ii) are described as variable volume, whereas system employing strategy (iv) is described as fixed volume. The used of strategy (iii) give a culture intermediate between the two extremes of variable and fixed volume. Since this invention is related to the production of thermostable lipase by recombinant *E.coli,* the most important factors in the design of these process include the secretion of the product, minimization of the degradation of the byproduct, avoiding media precipitation and preventing acetate accumulation, control the synthesis during the fermentation as well as maximizing the expression of the lipase gene and exploring the limits of *E.coli* culture density in order to achieve maximum productivity.

### Disclosure of the Invention

The present invention is a process for producing lipase by microorganisms on the industrial scale, wherein *Escherichia coli* microorganism harbouring lipase, preferably thermostable lipase, is fermented under batch or fed batch conditions, in a Luria Bertani (LB) broth supplemented with citric acid, (NH₄)₂SO₄, Na₂HPO₄.12H₂O, MgCl₂, NH₄Cl and glucose, using a trace metal solution containing hydrochloric acid, FeSO₄.7H₂O, ZnSO₄.7H₂0, CaCl₂.H₂0, MnSO₄.5H₂0, CuSO₄.5H₂O, AlCl.6H₂O, and H₃BO₃, and a feeding nutrient solution containing glucose, yeast extract, MgSO₄.H₂O, KH₂PO₄, Citric Acid, (NH₄)₂SO₄, Na₂HPO₄.12H₂O, MgCl₂, and NH₄Cl, at dissolved oxygen tension of 30-50 %, preferably 40 %, at temperature of 30-40 °C, preferably 37 °C, while maintaining the pH between 6 and 8, preferably at 6.5-7.5, more preferably at 7, with a glucose content of 15-25 g/L, preferably at 20 g/L, with isopropylthiogalactoside (IPTG), preferably 0.1 mM IPTG.

More preferably, the composition of the medium is LB broth 20.00 g/L, citric Acid 3.00 g/L, (NH₄)₂SO₄ 6.75 g/L, Na₂HPO₄.12H₂O 5.00 g/L, MgCl₂ 3.00 g/L, NH₄Cl 0.10 g/L and glucose 20.00g/L and distilled water.

More preferably, the composition of the trace metal solution is per ml of 5M acid hydrochloric FeSO₄.7H₂O 10.00 mg/ml, ZnSO₄.7H₂0 2.25 mg/ml, CaCl₂.H₂0 1.35 mg/ml, MnSO₄.5H₂0 0.50 mg/ml, CuSO₄.5H₂O 1.00 mg/ml, AlCl.6H₂O 0.30 mg/ml, and H₃BO₃ 0.20 mg/ml and distilled water.

More preferably, the feeding nutrient solution contains glucose 7.50 g/L, yeast extract 50.00 g/L, MgSO₄.H₂O 20.00 g/L, KH₂PO₄ 6.75 g/L, Citric Acid 3.00 g/L, (NH₄)₂SO₄ 6.75 g/L, Na₂HPO₄.12H₂O 5.00 g/L, MgCl₂ 3.00g/L, and NH₄Cl 0.10 g/L and distilled water.

### Brief Description of Figures

Figure 1: Effect of various values of dissolved oxygen tension (DOT) ranging from 10% to 60 % on mass and lipase activity
Figure 2 Effect of different temperature on lipase production during fed batch fermentation
Figure 3 Effect of different pH on lipase production during fed batch fermentation
Figure 4 Effect of different glucose concentration were added during fed batch fermentation
Figure 5 Effect of different IPTG concentration were induced during fed batch fermentation at A₆₀₀ₙₘ ≈ 0.5
Figure 6 Cultivation of 18 hours fed batch fermentation 200.0 L working volume using 300.0 L bioreactor size of E coli BL21 (DE3) with constant pH 7.0, temperature 37.0°C, stirrer speed 100 - 700 rpm, aeration 1.00 vvm and dissolved oxygen tension 40%. Glucose 20 g/L was added as carbon source. (x-axis: time (hours), left y-axis: %, °C, rpm, pH, right y-axis: L/min).

### Example:

*Escherichia coli* BL21 (DE3)plysS (pGEX /T1S) harbouring thermostable T1 lipase gene from *Geobacillus zalihae* strain growth on LB trybutyrin plate at 37 °C was used. Inoculum was prepared by inoculating one loop from clearing zone of overnight culture into 50 ml of the medium in 250 ml flask on a rotary shaker, 200 rpm at 37 °C. This maintained overnight culture then inoculated into 300 ml of the medium in a 2 L flask and cultivated with same shaker speed and temperature for 12 hours. Inoculum with the cell growth was used as starter, 10 % of fermentation. The medium contained Luria Bertani broth sterilized at 121 °C for 15 minutes and supplemented with ampicillin 50 mg/ml and choloroamphenicol 35 mg/ml.

For fermentation, the modified R-medium was used: LB broth 20.00 g/L, citric Acid 3.00 g/L, (NH₄)₂SO₄ 6.75 g/L, Na₂HPO₄.12H₂O 5.00 g/L, MgCl₂ 3.00 g/L, NH₄Cl 0.10 g/L and glucose 20.00g/L (or varied between 10-30 g/L). Glucose was sterilized separately. For fed batch regimen, trace metal solution was used, having the composition per ml of 5M acid hydrochloric FeSO₄.7H₂O 10.00 mg/ml, ZnSO₄.7H₂0 2.25 mg/ml, CaCl₂.H₂0 1.35 mg/ml, MnSO₄.5H₂0 0.50 mg/ml, CuSO₄.5H₂O 1.00 mg/ml, AlCl.6H₂O 0.30 mg/ml, and H₃BO₃ 0.20 mg/ml prepared with sterilized technique. The feeding nutrient solution contained glucose 7.50 g/L, yeast extract 50.00 g/L, MgSO₄.H₂O 20.00 g/L, KH₂PO₄ 6.75 g/L, Citric Acid 3.00 g/L, (NH₄)₂SO₄ 6.75 g/L, Na₂HPO₄.12H₂O 5.00 g/L, MgCl₂ 3.00g/L, and NH₄Cl 0.10 g/L. Glucose separately sterilized. Volume adjusted by distilled water.

The growth of *E. coli* can be limited by other nutritional requirement including carbon, nitrogen, phosphorus, sulfur, magnesium, potassium, iron, manganese, zinc, copper and some growth factor. When growing *E. coli* to low density, all required nutrients can be added initially into the basal broth. The LB allows the growth of *E. coli* in a temperature, pH, and oxygen controlled environment up to a cell density of 1 g/L. To accommodate the nutritional requirement of denser cultures, concentration media component must be increased and phosphorus, sulfur, and trace element must be added. Unfortunately, most media ingredients required for growth become inhibitory to *E. coli* when added at high concentrations. It is well established that nutrient such as glucose at concentration 50 g/L, ammonium at 3 g/L, iron at 1.15 g/L, magnesium at 8.7 g/L, phosphorus at 10 g/L and zinc at 0.038 g/L inhibit the *E. coli* growth.

The fermentation was performed in 3 L, 20 L and 200 L working volume with the parameters as shown in Table 2 below and with the procedures as follows:

### 3.0 L working volume optimization fed batch fermentation.

*Escherichia coli* BL21 (DE3)plysS (pGEX /T1S) harbouring thermostable T1 lipase gene from *Geobacillus zalihae* strain growth on LB trybutyrin plate at 37 °C. Inoculum was prepared by inoculating one loop from clearing zone of overnight culture into 50 ml of the medium in 250 ml flask on a rotary shaker, 200 rpm at 37 °C. This 50 ml overnight culture inoculated into 300 ml in 2 L flask contained Luria Bertani broth sterilized at 121 °C for 15 minutes and added with ampicillin 50 mg/ml and choloroamphenicol 35 mg/ml. The culture was further incubated at temperature 37 °C and shaker speed 200 rpm for 12 hours. This 300 ml culture then inoculate to 7.5 L bioreactor vessel containing 3.0 L working volume media. This 3.0 L working volume media which contain the modified R-medium: LB broth 60.00 g, citric Acid 9.00 g, (NH₄)₂SO₄ 20.25 g, Na₂HPO₄.12H₂O 15.00 g, MgCl₂ 9.00 g, NH₄Cl 0.30 g and glucose 60.00 g. Glucose sterilized separately, added with ampicillin 50 mg/ml and choloroamphenicol 35 mg/ml.

The composition of trace metal solution per ml of 5M acid hydrochloric contained FeSO₄.7H₂O 10.00 mg/ml, ZnSO₄.7H₂0 2.25 mg/ml, CaCl₂.H₂0 1.35 mg/ml, MnSO₄.5H₂0 0.50 mg/ml, CuSO₄.5H₂O 1.00 mg/ml, AlCl.6H₂O 0.30 mg/ml, and H₃BO₃ 0.20 mg/ml prepared with sterilized technique. This 3.0 L media sterilized at 121 °C for 15 minutes. This modified R-medium, glucose and trace metal solution added together for initial growth media. IPTG 0.100 mM added at OD=0.5 nm.

The feeding nutrient solution contained glucose 7.50 g/L, yeast extract 50.00 g/L, MgSO₄.H₂O 20.00 g/L, KH₂PO₄ 6.75 g/L, Citric Acid 3.00 g/L, (NH₄)₂SO₄ 6.75 g/L, Na₂HPO₄.12H₂O 5.00 g/L, MgCl₂ 3.00g/L, and NH₄Cl 0.10 g/L. Glucose separately sterilized. This feeding nutrient added during the beginning of stationary phase at 7-9 hours of fermentation.

The fermentation process will ended approximately at 18 hours before the lag phase and harvested for assay and proceed to spray dry process.

### 20.0 L working volume optimization fed batch fermentation.

*Escherichia coli* BL21 (DE3)plysS (pGEX /T1S) harbouring thermostable T1 lipase gene from *Geobacillus zalihae* strain growth on LB trybutyrin plate at 37 °C. Inoculum was prepared by inoculating one loop from clearing zone of overnight culture into 50 ml of the medium in 250 ml flask on a rotary shaker, 200 rpm at 37 °C. This 50 ml overnight culture inoculated into 300 ml in 2 L flask contained Luria Bertani broth sterilized at 121 C for 15 minutes and added with ampicillin 50 mg/ml, choloroamphenicol 35 mg/ml. This culture incubated at temperature 37 °C and shaker speed 200 rpm for 12 hours.

This 300 ml culture then inoculate into sterilize 3.0 L working volume contained Luria Bertani media at 121 °C for 15 minutes using 7.5 L bioreactor vessel and added with ampicillin 50 mg/ml and choloroamphenicol 35 mg/ml. This culture incubated at temperature 37 °C and stirrer speed 200 rpm for 12 hours.

Only 2.0 L culture from 3.0 L culture then inoculate into 30.0 L bioreactor vessel containing 20.0 L working volume media. This 20.0 L working volume contain modified R-medium LB broth: 400.00 g, citric Acid 60.00 g, (NH₄)₂SO₄ 26.75 g, Na₂HPO₄.12H₂O 100.00 g, MgCl₂ 60.00 g, NH₄Cl 2.00 g and glucose 400.00 g. Glucose sterilized separately. The composition of trace metal solution per ml of 5M acid hydrochloric contained FeSO₄.7H₂O 10.00 mg/ml, ZnSO₄.7H₂0 2.25 mg/ml, CaCl₂.H₂0 1.35 mg/ml, MnSO₄.5H₂0 0.50 mg/ml, CuSO₄.5H₂O 1.00 mg/ml, AlCl.6H₂O 0.30 mg/ml, and H₃BO₃ 0.20 mg/ml prepared with sterilized technique. This 20.0 L media sterilized at 121 °C for 15 minutes and added with ampicillin 50 mg/ml and choloroamphenicol 35 mg/ml.

This modified R-medium, glucose and trace metal solution added together for initial growth media. IPTG 0.100 mM added at OD=0.5 nm

The feeding nutrient solution contain glucose 7.50 g/L, yeast extract 50.00 g/L, MgSO₄.H₂O 20.00 g/L, KH₂PO₄ 6.75 g/L, Citric Acid 3.00 g/L, (NH₄)₂SO₄ 6.75 g/L, Na₂HPO₄.12H₂O 5.00 g, MgCl₂ 3.00g/L, and NH₄Cl 0.10 g/L. Glucose separately sterilized. This feeding nutrient added during the beginning of stationary phase at 7-9 hours of fermentation. The fermentation process will ended approximately at 18 hours before the lag phase and harvested for assay and proceed to spray dry process.

### 200.0 L working volume optimization fed batch fermentation

*Escherichia coli* BL21 (DE3)plysS (pGEX /T1S) harbouring thermostable T1 lipase gene from *Geobacillus zalihae* strain growth on LB trybutyrin plate at 37 °C. Inoculum was prepared by inoculating one loop from clearing zone of overnight culture into 50 ml of the medium in 250 ml flask on a rotary shaker, 200 rpm at 37 °C. This 50 ml overnight culture inoculated into 300 ml in 2 L flask contained Luria Bertani broth sterilized at 121 °C for 15 minutes and added with ampicillin 50 mg/ml and choloroamphenicol 35 mg/ml. This culture incubated at temperature 37 °C and shaker speed 200 rpm for 12 hours.

This 300 ml culture then inoculate to sterilize 3.0 L working volume Luria Bertani media at 121 °C for 15 minutes using 7.5 L bioreactor vessel and added with ampicillin 50 mg/ml and choloroamphenicol 35 mg/ml. This culture incubated at temperature 37 °C and stirrer speed 200 rpm for 12 hours.

Then, only 2.0 L culture from 3.0 L culture inoculate to 30.0 L bioreactor vessel containing 20.0 L working volume media. This 20.0 L media sterilize at 121 °C for 15 minutes and added with ampicillin 50 mg/ml and choloroamphenicol 35 mg/ml. This culture incubated at temperature 37 °C and stirrer speed 200 rpm for 12 hours.

This 20.0 L culture then inoculate to 300.0 L bioreactor vessel containing 200.0 L working volume media. This 200.0 L working volume contained the modified R-medium: LB broth 4000.00 g, citric Acid 600.00 g, (NH₄)₂SO₄ 1350.00 g, Na₂HPO₄.12H₂O 1000.00 g, MgCl₂ 600.00 g, NH₄Cl 20.00 g and glucose 4000.00 g. Glucose sterilized separately. The composition of trace metal solution per ml of 5M acid hydrochloric contained FeSO₄.7H₂O 10.00 mg/ml, ZnSO₄.7H₂0 2.25 mg/ml, CaCl₂.H₂0 1.35 mg/ml, MnSO₄.5H₂0 0.50 mg/ml, CuSO₄.5H₂O 1.00 mg/ml, AlCl.6H₂O 0.30 mg/ml, and H₃BO₃ 0.20 mg/ml prepared with sterilized technique. This 200.0 L media sterilized at 121 °C for 15 minutes and added with ampicillin 50 mg/ml and choloroamphenicol 35 mg/ml.

This modified R-medium, glucose and trace metal solution added together for initial growth media. IPTG 0.100 mM added at OD=0.5 nm

The feeding nutrient solution contain glucose 7.50 g/L, yeast extract 50.00 g/L, MgSO₄.H₂O 20.00 g/L, KH₂PO₄ 6.75 g/L, Citric Acid 3.00 g/L, (NH₄)₂SO₄ 6.75 g/L, Na₂HPO₄.12H₂O 5.00 g/L, MgCl₂ 3.00g/L, and NH₄Cl 0.10 g/L. Glucose separately sterilized. This feeding nutrient added during the beginning of stationary phase at 7-9 hours of fermentation. The fermentation process will ended approximately at 18 hours before the lag phase and harvested for assay and proceed to spray dry process.

### Fed-batch fermentation parameter

Bioreactor are parameter setting: dissolved oxygen tension 40 %, temperature 37 °C, pH 7.0, stirrer speed 200-800 rpm, cascade system on and air flow rate at 1.0 vvm.

Cell growth was monitored by measuring the optical density, OD at 600 nm using sterile medium as a blank by UV spectrophotometer.

Lipase activity was assessed using the Kwon and Rhee [Kwon, D. Y. and Rhee, J. S. 1986. A simple and rapid colorimetric method for determination of free fatty acid for lipase assay. Journal of American Oil Chemist Society 63: 69 -92] colorimetric method with slight modification by changing only the buffer. Olive oil was emulsified with glycine-NaOH ph 9 (1:1 v/v) with a magnetic stirrer. Then, 2.5 ml of oil emulsion aliquot in the reaction bottle, followed with 20.00 µl of 0.02 mM CaCl₂. The reaction was triggered by the addition of 10.00 µl of crude enzyme. Reaction bottle was then incubated in a water bath shaker with stirring speed 200 rpm and temperature 70°C for 30 minutes. Reaction stopped by adding 1.00 ml 6N HCl and isooctane 5.00 ml, followed by vortex the reaction mixture for 30 seconds. 4 ml of the upper layer removed to test tube containing 1.00 ml cupric acetate pyridine for analysis. Free fatty acids dissolved in isooctane were measured using spectrophotometer at 715 nm. Lipase activity was determined by measuring the amount of free fatty acids released base on the standard curve of oleic acid.

Crude lipase was prepared as follows: 15 ml of culture collected from the bioreactor and harvested by centrifugation 10,000 rpm for 10 minutes at 4°C. Remove the media and all the pellets resuspended in 15 ml of gylcine-NaOH pH 9 in low temperature. The cell pellets were sonicated and centrifuge again at 10,000 rpm for 10 minutes at 4°C. Then, the supernatants contain crude lipase and stored at 4°C. For a spray-dried lipase, 100 g pellet was resuspended in 1000 ml of glycine-NaOH pH 9 in low temperature. The cell pellets were sonicated at 10 minutes with 30 second interval and centrifuged at 10,000 rpm for 10 minutes at 4°C. Then, the supernatants contain crude lipase spray dried using spray dryer at temperatures ranging from 60° C to 110° C, aspirator at 100 %, pump 50 %, nozzle cleaner at 5 and air flow rate at 60 L/min. The powder kept at 4 °C chiller.

### Results:

### 1) 3.0 L working volume fed batch fermentation of dissolved oxygen tension (DOT) optimization

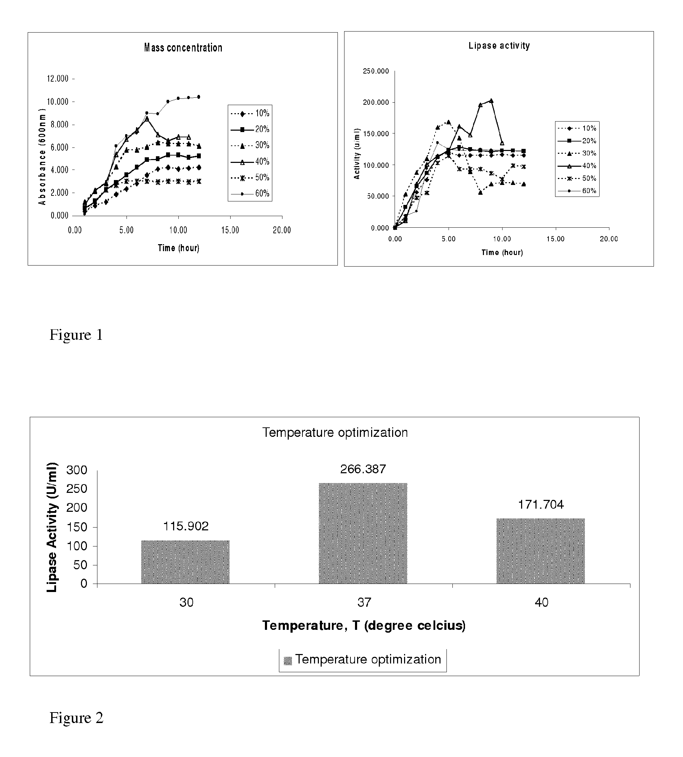

Figure 1 shows the effect of different dissolved oxygen tension (DOT) ranging from 10% to 60 % on mass and lipase activity.

In aerobic process oxygen must be continuously supplied in order to achieve acceptable productivities. Since the role of oxygen in microorganism growth and its metabolism is of vital importance, both the oxygen consumption by the cell and the dissolved oxygen transfer (DOT) into the system has to be understood. The main function of a properly designed bioreactor is to provide a controlled environment and a concentration of nutrients such as dissolved oxygen must be sufficient to achieve optimal growth or optimal product formation in a particular bioprocess. Dissolved oxygen in the broths is the result of a balance of its consumption rate in the cells, and the rate of oxygen transfer from the gas to the liquid phase. Monitoring dissolved oxygen in the broth is mandatory because often oxygen becomes the factor governing the metabolic pathways in microbial cells. In this work the dissolved oxygen tension (DOT) in different percentage of concentration is examined.

Oxygen is a key substrate employed for growth, maintenance and in other metabolic routes, including product synthesis. Due to its low solubility in broths, which are usually aqueous solutions, oxygen must be continuously provided by a gas phase, and thus the knowledge of oxygen transfer rate (OTR) is needed for bioreactor design and scale-up. The concentration of dissolved oxygen in the broth, a suspension of respiring microorganisms, depends on the OTR from the gas to the liquid phase, and on the rate of its consumption by the microorganism, the oxygen uptake rate (OUR). Both oxygen mass transfers from the gas to the liquid phase and its consumption by microorganism has a decisive importance, since in many processes oxygen transfer is the controlling step for the microbial growth, and can affect the evolution of bioprocesses. The results show that the optimum dissolved oxygen tension is 30-40 %.

### 2) 3.0 L working volume fed batch fermentation of temperature optimization

Figure 2 shows the effect of different temperature on lipase production during fed batch fermentation.

Temperature ranging from 30, 37 and 40 were tested to study the effect of temperature on lipase production during fed batch fermentation. The highest amount of lipase production was detected at 37 °C at 266.387 U/ml. However, the lipase production was not too high during 40 °C and the 30 °C fed batch fermentation shown a lowest lipase production at 115.902 U/ml. The low production of lipase can be answered by the aggregation reaction being overcome at higher temperature due to strong hydrophobic interaction of expressed protein form insoluble aggregates.

### 3) 3.0 L working volume fed batch fermentation of pH optimization

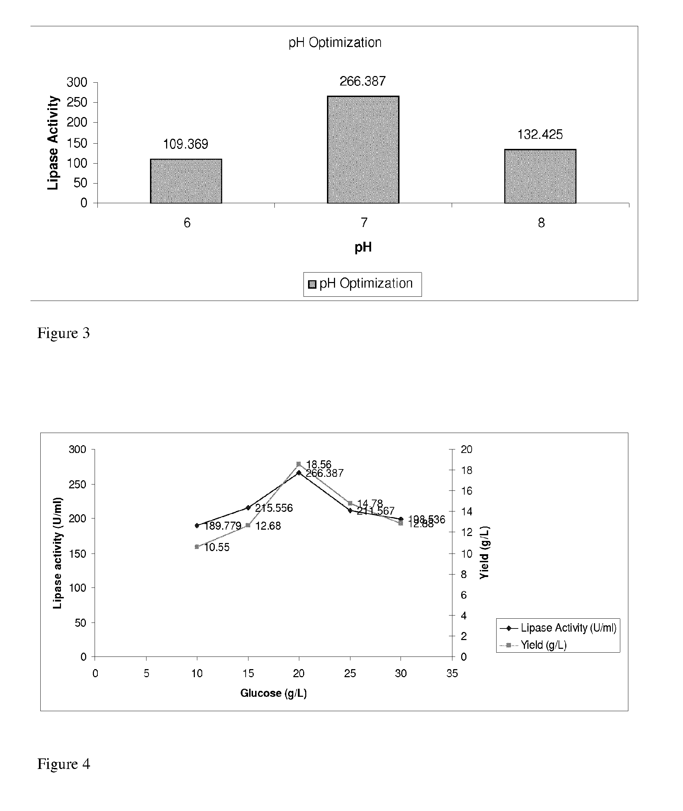

Figure 3 shows the effect of different pH on lipase production during fed batch fermentation.

Biotechnological processes are usually conducted in fed-batch cultivation mode with active pH-monitoring and control. In contrast, shake flask experiments are usually conducted in batch mode without active pHcontrol, but with high initial buffer concentrations to prevent excessive pH-drifts during the cultivation. High buffer and substrate concentrations in small-scale cultivation media lead to low water activity and high osmolarity. These conditions may inhibit the growth of microorganisms. For example, since the optimal osmolarity of a medium to cultivate Escherichia coli is approximately 0.3 Osmol/L, increasing or decreasing the osmolarity results in reduced bacterial growth rates. Consequently, the application of high buffer and substrate concentrations in the screening for optimal production strains might handicap microorganisms with high potential but low osmotolerance.

The pH-value is an important parameter for cultivating microorganisms. The best pH-range for cultivating e.g. Escherichia coli is 6.5-7.5, and varies with temperature. The metabolic activity of the cultivated microorganisms influences the pH-value of the surrounding medium in different ways. As microorganisms cultivated in mineral media with glucose or glycerol as carbon source and ammonium salt as nitrogen source, produce one proton [H+] per consumed molecule of ammonium, the pH of the medium decreases during the cultivation. Furthermore, under oxygen-limiting conditions and in the overflow metabolism, E. coli produce acetate. This also decreases the pH-value. Once the glucose is depleted and the pH in the medium is not too low for growth of the microorganisms, the accumulated acetate is consumed as a second carbon source and the pH subsequently increases. Moreover, metabolically generated bicarbonate ions accumulate in the medium and decrease the pH-value. Therefore, the pH-value changes significantly during fermentations. Consequently, a pH-control is absolutely vital to maintain physiological pH values during microbial cultivation.

### 4) 3.0 L working volume fed batch fermentation of glucose optimization

Figure 4 shows the effect of different glucose concentration were added during fed batch fermentation.

Complex media such as LB (Luria Bertani) have been successfully used to grow Escherichia coli in small-scale cultures. Since the development of recombinant DNA technology and the production of heterologous proteins, it has become increasingly important to optimize growth and expression. However, the physiological implications of small scale culture conditions, as opposed to fermentation processes in stirred bioreactors, have not been studied in detail. Successful expression of proteins in a shake flask cannot easily be up-scaled, if cultivation parameters are not well characterized. Moreover, unfavorable conditions during screening for a production strain may lead to selection of a strain that is unsuitable for large-scale fermentations.

Supplementing LB medium with glucose under the same shaking conditions led to a decrease of respiratory activity corresponding to a rapid drop in pH. Overflow metabolism, resulting in the production of lactic and acetic acid was responsible for this adverse effect. The small amount of glucose that was metabolized was primarily converted to acetic acid. The final cell density was lower than that formed in LB medium. The maximum ammonium concentration was also two times lower than that in LB medium. Because high concentrations of acetate and lactate are toxic especially at low pH, LB-glucose was buffered with phosphate in fed batch and same way as for LB medium in batch with the same shaking condition. Buffering significantly improved the respiratory activity, but the OTR did not reach the oxygen transfer capacity. Glucose usually consumed within 12 h. However, overflow metabolism still occurred. While acetate accumulated, counter for almost 50% of the original carbon source added to the medium.

### 5) 3.0 L working volume fed batch fermentation of IPTG concentration optimization

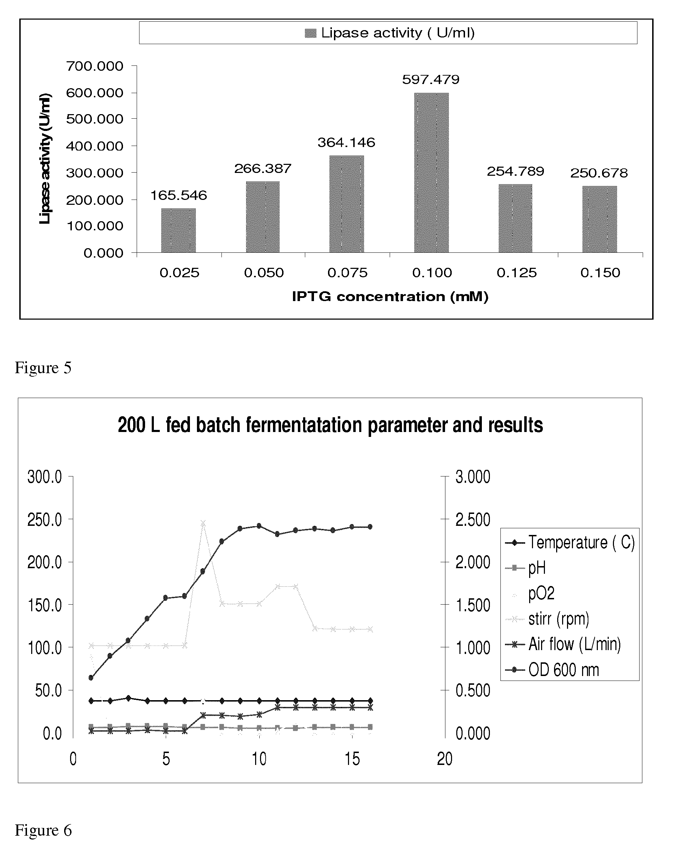

Figure 5 shows the effect of different IPTG concentration were induced during fed batch fermentation at A₆₀₀ₙₘ ≈ 0.5.

Isopropylthiogalactoside (IPTG) at 0.100 mM was the optimum concentration for lipase production during the fed batch fermentation at 597.479 U/ml compared to 0.025 and 0.150 mM at 165.546 U/ml and 250.678 U/ml respectively. The well homogenized condition between the media, air, oxygen supply and the inducer produced a higher lipase activity. A cascade system was applied during the fed batch fermentation and the stirrer speed was set at 100 to 900 rpm to maintain the 40% dissolved oxygen. Indirectly, it was created a very well mixing rate and the inducer was added at the early of log phase. This is the benefit of bioreactor where the parameter can be observed and the suitable environment can be prepared to the sample.

### 6) 200.0 L working volume media fed batch fermentation manual parameter monitoring

Figure 6 shows the cultivation of 18 hours fed batch fermentation 200.0 L working volume using 300.0 L bioreactor size of E coli BL21 (DE3) with constant pH 7.0, temperature 37.0°C, stirrer speed 100 - 700 rpm, aeration 1.00 vvm and dissolved oxygen tension 40%. Glucose 20 g/L was added as carbon source.

**Table 1: Comparison of cell concentration (g), optical density, yield (g/L) and lipase activity (U/ml) between 3.0 L, 20.0 L batch and fed-batch and 200.0 L fed-batch fermentation**

| Type | Cell concentration (g) | Optical Density (600nm) | Yield (g/L) | Lipase activity (U/ml) |
|---|---|---|---|---|
| 3.0 L working volume | | | | |
| Batch | 33.076 | 14.894 | 5.39 | 202.194 |
| Fed Batch | 79.817 | 26.600 | 18.56 | 266.387 |

| 20.0 L working volume | | | | |
|---|---|---|---|---|
| Batch | 21.004 | 6.357 | 2.142 | 68.373 |
| Fed Batch | 67.453 | 2.134 | 1.483 | 132.372 |

| 200.0 L working volume | | | | |
|---|---|---|---|---|
| Fed batch | 3841 | 18.457 | 193.402 | 178.889 |

**Table 2: Parameters of the fermentations**

| | | | |
|---|---|---|---|
| Process (working volume) | 3.0 L | 20.0 L | 200.0 L |
| Vessel (maximum volume) | 7.5 L | 30.0 L | 300.0 L |
| Fermentation | 1. Batch 2. Fed batch | 1. Batch 2. Fed batch | 1. Fed batch |
| Cultivation period | 18 hours | 18 hours | 18 hours |
| Media | The modified R-medium | The modified R-medium | The modified R-medium |
| Carbon source | Glucose | Glucose | Glucose |
| Trace element | Formulized | Formulized | Formulized |
| Feeding nutrient | Formulized | Formulized | Formulized |
| Sterilization | Laboratory Autoclave | *In-situ* | *In-situ* |
| Media Optimization | Done | Done | Done |
| Chloroamphenicol | 35 mg/ml | 35 mg/ml | 35 mg/ml |
| Ampicillin | 50 mg/ml | 50 mg/ml | 50 mg/ml |
| Dissolved Oxygen Optimization | 40% | 40% | 40% |
| Temperature Optimization | 37 °C | 37 °C | 37 °C |
| Temperature tested | 28 - 40 °C | 28 - 40 °C | 28 - 40 °C |
| pH Optimization | 7 | 7 | 7 |
| pH tested | 6-9 | 6-9 | 6-9 |

| Inducer | IPTG | IPTG | IPTG |
|---|---|---|---|
| Inducer concentration Optimization | 0.100 mM | 0.100 mM | 0.100 mM |
| Inducer concentration tested | 0.025 - 0.125 mM | 0.025 - 0.125 mM | - |
| kLa tested | Done | Done | Done |
| Air flow rate | 0 - 1.0 vvm | 0 -1.0 vvm | 0 -1.0 vvm |
| Cascade system | ON | ON | ON |
| Without Cascade | Done | Done | Done |
| Cell separator | High speed centrifuge | High speed centrifuge | Continues centrifuge |
| Cell sonicator | Industrial Ultra sonicator | Industrial Ultra sonicator | Industrial Ultra sonicator |
| Sonication Buffer | Added | Added | Added |
| Lipase assay | Done | Done | Done |
| Protein Assay | Done | Done | Done |
| Protein Identification | SDS page | SDS page | SDS page |
| Final Product | Crude T1 Lipase | Crude T1 Lipase | Crude T1 Lipase |
| Spray dried | Done | Done | Done |
| Packaging | - | - | Done |
| Final Product Storage | - | - | 4 °C chiller |
| Final Product Tested | - | - | Done |

## Claims

1. A process for producing lipase by microorganisms, wherein *Escherichia coli* microorganism harbouring lipase, preferably thermostable lipase, is fermented under batch or fed batch conditions, in a Luria Bertani (LB) broth supplemented with citric acid, (NH₄)₂SO₄, Na₂HPO₄.12H₂O, MgCl₂, NH₄Cl and glucose, using a trace metal solution containing hydrochloric acid, FeSO₄.7H₂O, ZnSO₄.7H₂0, CaCl₂.H₂0, MnSO₄.5H₂0, CuSO₄.5H₂O, AlCl.6H₂O, and H₃BO₃, and a feeding nutrient solution containing glucose, yeast extract, MgSO₄.H₂O, KH₂PO₄, Citric Acid, (NH₄)₂SO₄, Na₂HPO₄.12H₂O, MgCl₂, and NH₄Cl, at dissolved oxygen tension of 30-50 %, at temperature of 30-40 °C, while maintaining the pH between 6 and 8, with a glucose content of 15-25 g/L, with isopropylthiogalactoside.

2. The process of claim 1, wherein the dissolved oxygen tension is 40 %.

3. The process of claim 1, wherein the temperature is 37 °C.

4. The process of claim 1, wherein the pH is 6.5-7.5.

5. The process of claim 1, wherein the glucose content is 20 g/L.

6. The process of claim 1, wherein 0.1 mM isopropylthiogalactoside is used.

7. The process of claim 1, wherein the composition of the medium is LB broth 20.00 g/L, citric Acid 3.00 g/L, (NH₄)₂SO₄ 6.75 g/L, Na₂HPO₄.12H₂O 5.00 g/L, MgCl₂ 3.00 g/L, NH₄Cl 0.10 g/L and glucose 20.00g/L and distilled water.

8. The process of claim 1, wherein the composition of the trace metal solution is per ml of 5M acid hydrochloric FeSO₄.7H₂O 10.00 mg/ml, ZnSO₄.7H₂0 2.25 mg/ml, CaCl₂.H₂0 1.35 mg/ml, MnSO₄.5H₂0 0.50 mg/ml, CuSO₄.5H₂O 1.00 mg/ml, AlCl.6H₂O 0.30 mg/ml, and H₃BO₃ 0.20 mg/ml and distilled water.

9. The process of claim 1, wherein the feeding nutrient solution contains glucose 7.50 g/L, yeast extract 50.00 g/L, MgSO₄.H₂O 20.00 g/L, KH₂PO₄ 6.75 g/L, Citric Acid 3.00 g/L, (NH₄)₂SO₄ 6.75 g/L, Na₂HPO₄.12H₂O 5.00 g/L, MgCl₂ 3.00g/L, and NH₄Cl 0.10 g/L and distilled water.
